# EUROPÄISCHE PATENTANMELDUNG

(11) **EP 3 028 763 A1**
(43) Veröffentlichungstag der Anmeldung: **08.06.2016**
(21) Anmeldenummer: 14196648.1
(22) Anmeldetag: 05.12.2014
(51) Int. Cl.: B01J 19/00, C07C 5/333, C07C 11/06, C10G 45/00

(54) **Verfahren und Anlage zur Gewinnung von Propylen**

(71) Anmelder: Linde Aktiengesellschaft, 80331 München (DE)
(72) Erfinder: Höfel, Dr. Torben, 81543 München (DE); Stahl, Bernhard, 81379 München (DE); Conradt, Jörg, 80639 München (DE); Bölt, Heinz, 82515 Wolfratshausen (DE)
(74) Vertreter: Frommberger, Moritz

(57) **Zusammenfassung**

Es wird ein Verfahren (100) zur Gewinnung von Propylen vorgeschlagen, bei dem aus Fluid eines Propan enthaltenden Dehydrierungseinsatzstroms (A) und eines Wasserdampfstroms (B) in einem Dehydrierungsschritt (101) ein Wasserdampf, Wasserstoff sowie Kohlenwasserstoffe mit einem bis vier Kohlenstoffatomen, darunter Propylen, enthaltender, erwärmter Dehydrierungsabstrom (C) gebildet wird, Fluid des Dehydrierungsabstroms (C) in einem Waschschritt (105) in direkten Kontakt mit einem Wasserstrom (Z) gebracht und abgekühlt und dabei ein erwärmter und überwiegend Wasserdampf des Dehydrierungsabstroms (C) und Wasser des Wasserstroms (Z) enthaltender Kondensatwasserstrom (D) sowie ein an Wasserdampf abgereicherter, zumindest Wasserstoff und Kohlenwasserstoffe mit einem bis vier Kohlenstoffatomen enthaltender Waschabstrom (F) gebildet wird, und zumindest unter Verwendung von Fluid des Waschabstroms (F) ein Kohlenwasserstoffe mit drei und vier Kohlenstoffatomen enthaltender, an Wasserstoff, Kohlenwasserstoffen mit einem und zwei Kohlenstoffatomen und Propylen armer, flüssiger Verdampfungseinsatzstrom (V) gebildet und verdampft wird, wobei das zu Bildung des Verdampfungseinsatzstroms (V) verwendete Fluid des Waschabstroms (F) unter Verwendung einer Deethanizersäule (11, 21) gebildet wird, in die zumindest ein aus dem Waschabstrom (F) gebildetes Fluid eingespeist wird. Vorgesehen ist, dass Beheizung eines Sumpfs der Deethanizersäule (11, 21) und zur Verdampfung des Verdampfungseinsatzstroms (V) Wärme des Kondensatwasserstroms (D) verwendet wird. Eine entsprechende Anlage ist ebenfalls Gegenstand der vorliegenden Erfindung.

## Beschreibung

Die Erfindung betrifft ein Verfahren und eine Anlage zur Gewinnung von Propylen gemäß den Oberbegriffen der unabhängigen Patentansprüche.

### Stand der Technik

Verfahren zur Dehydrierung von Alkanen, insbesondere von Propan zu Propylen, sind bekannt und beispielsweise im Artikel "Propene" in Ullmann's Encyclopedia of Industrial Chemistry, Onlineausgabe, 15. Juni 2000, DOI 10.1002/14356007.a22_211, Abschnitt 4.3., "Propane Dehydrogenation", beschrieben.

Zur Dehydrierung von Propan zu Propylen werden beispielsweise Reaktoren verwendet, die mittels Brennern beheizte Brennkammern aufweisen, durch welche mehrere Reaktionsrohre geführt sind. Die Reaktionsrohre werden von außen durch die Brenner beheizt. Die Reaktionsrohre enthalten einen Katalysator auf einem geeigneten Träger. Geeignete Katalysatoren sind beispielsweise bei Bölt, H., "Dehydrierung von Propan/Butan", Linde Berichte aus Technik und Wissenschaft 66/1991, beschrieben. Durch die Reaktionsrohre wird das zu dehydrierende Propan bzw. ein propanreicher Strom zusammen mit Dampf, sogenanntem Prozessdampf, geführt. Zuvor erfolgt typischerweise eine Vorwärmung mittels Abwärme. Ein dem Reaktor bzw. entsprechenden Reaktionsrohren entnommenes Gasgemisch, nachfolgend auch als Produktstrom bezeichnet, wird einer Produktaufbereitung zugeführt.

Die Dehydrierung von Propan zu Propylen ist bei der Wahl geeigneter Katalysatoren und Reaktionsbedingungen hochselektiv und kann bei über 90% liegen. Dennoch entstehen immer auch geringe Mengen an Nebenprodukten in Form von Wasserstoff, Methan sowie Kohlenwasserstoffen mit zwei und mehr als drei Kohlenstoffatomen. Ferner finden sich in einem entsprechenden Produktstrom immer auch größere Mengen an nicht zu Propylen umgesetztem Propan und Prozessdampf.

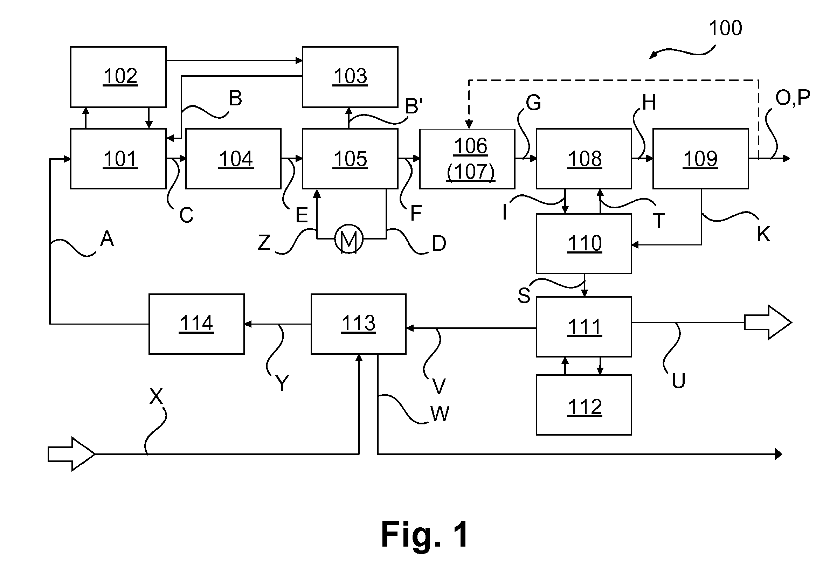

Wie auch unter Bezugnahme auf die beigefügte Figur 1 erläutert, umfassen gängige Verfahren zur Gewinnung von Propylen daher, den Produktstrom zunächst zu kühlen und Wasser aus diesem abzuscheiden. Der auf diese Weise teilweise von Wasser befreite Produktstrom wird dann einer weiteren Aufreinigung unterworfen und zumindest teilweise in seine Kohlenwasserstoffkomponenten aufgetrennt.

Im Rahmen einer entsprechenden Trennsequenz wird typischerweise unter Abtrennung anderer Komponenten ein flüssiger Strom gebildet, der lediglich noch Kohlenwasserstoffe mit drei, vier und gegebenenfalls mehr Kohlenstoffatomen in nennenswertem Umfang enthält, der jedoch arm an oder (im Wesentlichen) frei von Wasserstoff, Methan, Kohlenwasserstoffen mit zwei Kohlenstoffatomen und Propylen ist. Es handelt sich damit um einen sogenannten C3- oder C3plus-Strom (siehe unten), aus dem das enthaltene Propylen bereits (zumindest weitgehend) abgetrennt wurde. Dieser Strom enthält noch größere Mengen an Propan, das vorteilhafterweise zurückgewonnen und zur Dehydrierung zurückgeführt wird.

Hierzu werden typischerweise zunächst die Kohlenwasserstoffe mit vier und gegebenenfalls mehr Kohlenstoffatomen von den Kohlenwasserstoffen mit drei Kohlenstoffatomen getrennt. Diese sogenannte C3/C4-Trennung erfolgt mittels einer Destillationssäule, in die der aufzutrennende Strom typischerweise zusammen mit einem von extern zugeführten propanreichen Strom, also einem Propanfrischeinsatz, eingespeist wird. Es erweist sich dabei als energetisch vorteilhaft, zumindest den aufzutrennenden Strom, der zunächst flüssig vorliegt, vor dem Einspeisen in die Destillationssäule zu verdampfen. Auch der Propanfrischeinsatz sollte zweckmäßigerweise verdampft werden, wenn er in flüssiger Form vorliegt.

Gängige Verfahren, die eine derartige Verdampfung umfassen, sind jedoch häufig aus energetischer bzw. ökonomischer Sicht nicht vollständig zufriedenstellend. Die Erfindung stellt sich daher die Aufgabe, herkömmliche Verfahren und Anlagen zur Gewinnung von Propylen zu verbessern.

### Offenbarung der Erfindung

Diese Aufgabe wird durch ein Verfahren und eine Anlage zur Gewinnung von Propylen mit den Merkmalen der unabhängigen Patentansprüche gelöst. Bevorzugte Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ±1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Flüssige und gasförmige Ströme können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält. Ist hier beispielsweise vereinfacht von "Propan" die Rede, sei darunter ein Strom verstanden, der reich an Propan ist, jedoch nicht ausschließlich aus Propan bestehen muss. Ist hier und im Folgenden davon die Rede, dass "aus Fluid" eines Stroms ein weiterer Strom gebildet wird, kann hierunter sowohl fallen, dass der weitere Strom aus der gesamten Menge des Fluids des jeweiligen Ausgangsstroms gebildet wird, aber auch, dass der weitere Strom nur aus einem Teil hiervon gebildet oder zusätzliches Fluid verwendet wird.

Vorliegend können zur Trennung von Kohlenwasserstoffströmen insbesondere Destillationssäulen und Absorptionskolonnen zum Einsatz kommen. Zur Auslegung und Ausgestaltung entsprechender Vorrichtungen sei auf einschlägige Lehrbücher verwiesen (siehe beispielsweise K. Sattler: Thermische Trennverfahren. Grundlagen, Auslegung, Apparate. Weinheim: Wiley-VCH, 3. Auflage 2001).

Gängige Verfahren zur Trennung von Produktströmen aus Verfahren zur Herstellung von Kohlenwasserstoffen umfassen die Bildung einer Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (und konventionsgemäß unter Umständen auch Wasserstoff, dann auch "C1minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für die "C5-Fraktion" und die höheren Fraktionen. Mehrere solcher Fraktionen können zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

### Vorteile der Erfindung

Die vorliegende Erfindung geht von einem an sich bekannten Verfahren zur Gewinnung von Propylen aus, bei dem aus Fluid eines Propan enthaltenden Dehydrierungseinsatzstroms und eines Wasserdampfstroms (dem erwähnten Prozessdampf) in einem Dehydrierungsschritt ein Wasserdampf, Wasserstoff sowie Kohlenwasserstoffe mit einem bis vier (ggf. auch mehr) Kohlenstoffatomen, darunter Propylen, enthaltender und erwärmter Dehydrierungsabstrom gebildet wird. Die Zugabe von Wasserdampf erfolgt zur Verdünnung des Propans bzw. zur Verringerung seines Partialdrucks, wodurch Reaktionsbedingungen geschaffen werden, die die Selektivität und Ausbeute der Dehydrierung erhöhen. Der Dehydrierungsabstrom kann, wie auch unter Bezugnahme auf die beigefügten Zeichnungen näher erläutert, einem oder mehreren Aufbereitungsschritten unterworfen werden, beispielsweise einer Vorkühlung und einer Kondensation schwererer Verbindungen.

Fluid des Dehydrierungsabstroms wird anschließend in einem Waschschritt in direkten Kontakt mit einem Wasserstrom gebracht, also einer sogenannten Wasserwäsche bzw. einem Wasserquench unterworfen. Das Fluid des Dehydrierungsabstroms wird dabei abgekühlt, wobei ein erwärmter und überwiegend Wasserdampf des Dehydrierungsabstroms in kondensierter Form sowie Wasser des Wasserstroms enthaltender Kondensatwasserstrom einerseits, sogenanntes Quenchwasser, sowie ein an Wasserdampf abgereicherter, zumindest Wasserstoff und Kohlenwasserstoffe mit einem bis vier Kohlenstoffatomen enthaltender Waschabstrom gebildet wird. Der Waschabstrom weist dabei typischerweise noch die wesentlichen Kohlenwasserstoffkomponenten des Dehydrierungsabstroms auf.

Wie bereits teilweise erläutert, umfassen Trennsequenzen zur Aufbereitung von Dehydrierungsabströmen aus Dehydrierungsverfahren typischerweise, einen Kohlenwasserstoffe mit drei und vier (ggf. auch mehr) Kohlenstoffatomen enthaltenden, jedoch an Wasserstoff, Kohlenwasserstoffen mit einem oder zwei Kohlenstoffatomen sowie Propylen abgereicherten und daher an den genannten Verbindungen armen oder (im Wesentlichen) freien, flüssigen Strom zu bilden. Dieser Strom, hier als Verdampfungseinsatzstrom bezeichnet, wird verdampft und dient anschließend als Einsatzstrom für eine C3/C4-Trennung in der, wie zuvor erläutert, Kohlenwasserstoffe mit vier (und mehr, falls vorhanden) Kohlenstoffatomen zumindest teilweise abgetrennt werden. Wie erläutert, wird entsprechendes Fluid vorteilhafterweise verdampft, um die anschließende C3/C4-Trennung energieeffizient gestalten zu können. Der Verdampfungseinsatzstrom kann neben Fluid des Waschabstroms auch weiteres Fluid, beispielsweise eines zusätzlichen, von extern zugeführten propanreichen Stroms, also eines Frischeinsatzes, umfassen. Erfindungsgemäß ist nun vorgesehen, dass zur Verdampfung des Verdampfungseinsatzstroms Wärme des Kondensatwasserstroms verwendet wird. Wie bereits erläutert, wird ein Dehydrierungsabstrom typischerweise bei vergleichsweise niedrigem Druck in Direktkontakt mit einem Wasserstrom in einer Wasserwäsche abgekühlt, wodurch zuvor eingesetzter Prozessdampf zumindest teilweise kondensiert wird. Das dabei anfallende Quenchwasser, das im Rahmen der vorliegenden Anmeldung als Kondensatwasserstrom bezeichnet wird, wird hierdurch bis auf eine Temperatur von typischerweise 94 °C aufgeheizt. Die Temperatur des Kondensatwasserstroms reicht damit zur Verdampfung des Verdampfungseinsatzstroms aus, da dieser typischerweise bei vergleichsweise geringem Druck von beispielsweise ca. 17 bar vorliegt. Bei einem entsprechenden Druck erfolgt eine Verdampfung bereits bei ca. 50 °C.

Das erfindungsgemäße Verfahren umfasst ferner, Fluid des Waschabstroms unter Verwendung einer beheizten Destillationssäule einer Destillation zu unterwerfen. Es handelt sich hierbei um eine Destillationssäule an sich bekannter Art, die im Folgenden der Eindeutigkeit halber und zur besseren Unterscheidung von der zur C3/C4-Trennung verwendeten Destillationssäule als "Deethanizersäule" bezeichnet wird. Das in die Deethanizersäule eingespeiste Fluid des Waschabstroms wird gegenüber dem Waschabstrom vorteilhafterweise an leichteren Komponenten, d.h. insbesondere an Methan und Wasserstoff, abgereichert. Zur Abreicherung eines entsprechenden Fluids an Methan und Wasserstoff können voran- und teilweise parallel geschaltete Trenn-und Kondensationsschritte verwendet werden, wie sie insbesondere unter Bezugnahme auf die Figuren 1 bis 3 erläutert werden.

Die Deethanizersäule, d.h. insbesondere ihr Sumpfverdampfer, wird erfindungsgemäß ebenfalls unter Verwendung von Wärme des Kondensatwasserstroms beheizt. Die Vorteile einer entsprechenden Beheizung entsprechen dabei zumindest teilweise jenen, die bereits unter Bezugnahme auf die Verdampfung des Verdampfungseinsatzstroms erläutert wurden. Ein weiterer großer Vorteil ist eine deutliche Verringerung des Foulingrisikos in einem entsprechenden Sumpfverdampfer, da durch die Verwendung des Kondensatwasserstroms eine deutlich geringere Temperaturdifferenz vorliegt als bei der Verwendung herkömmlicher Wärmemedien, beispielsweise Dampf.

Der wesentliche Vorteil der vorliegenden Erfindung ist dabei insbesondere der, dass zur Verdampfung des Verdampfungseinsatzstroms und zum Betrieb der Deethanizersäule keine externe Wärmequelle erforderlich ist, was zu einer signifikanten Reduzierung der Betriebskosten des Verfahrens führen kann. Ferner wird im Rahmen der vorliegenden Erfindung der Kondensatwasserstrom weiter abgekühlt, wodurch weniger Wärme durch Kühlwasser oder Luftkühlung an die Umwelt abgeführt werden muss.

Vorteilhafterweise werden zum Verdampfen des Verdampfungseinsatzstroms und zum Beheizen der Deethanizersäule jeweils ein Wärmetauscher verwendet, wie bereits angesprochen. Fluid des Kondensatwasserstroms wird dabei durch den zum Verdampfen des Verdampfungseinsatzstroms und den zum Beheizen der Deethanizersäule verwendeten Wärmetauscher geführt. Gemäß einer besonders vorteilhaften Ausführungsform der Erfindung ist vorgesehen, das Fluid des Kondensatwasserstroms vor dem Führen durch den zum Verdampfen des Verdampfungseinsatzstroms verwendeten Wärmetauscher zunächst durch den zum Beheizen der Deethanizersäule verwendeten Wärmetauscher zu führen. Hierbei ergibt sich ein besonderer Synergieeffekt in Anlagen zur Propandehydrierung, wie im folgenden noch näher erläutert wird.

In typischen Olefinanlagen, d.h. beispielsweise Steamcrackern mit nachgeschalteten Trenneinheiten, kann eine Deethanizersäule nicht mit einem Kondensatwasserstrom betrieben werden, da entweder die Drücke zu hoch sind (beispielsweise mehr als ca. 30 bar betragen) und/oder Fluide mit nennenswerten Anteilen an Kohlenwasserstoffen mit vier und mehr Kohlenstoffatomen im Sumpf vorliegen. Somit sind die Sumpftemperaturen zu hoch und können durch die erwähnte Temperatur des Kondensatwasserstroms nicht erreicht werden. Bei der Propandehydrierung werden der Dehydrierungsabstrom bzw. die daraus gebildeten Ströme jedoch typischerweise auf geringere Drücke verdichtet, so dass im Sumpf einer Deethanizersäule nur ein Druck von typischerweise ca. 28 bar vorliegt. Außerdem sind die Konzentrationen von Kohlenwasserstoffen mit vier und mehr Kohlenstoffatomen im Sumpf vergleichsweise gering. Diese Bedingungen korrespondieren mit einer Temperatur von ca. 70 °C, die durch einen Kondensatwasserstrom einfach erreicht werden kann. Werden der zur Beheizung der Deethanizersäule verwendete Wärmetauscher und der zum Verdampfen des Verdampfungseinsatzstroms verwendete Wärmetauscher in Reihe geschaltet, ergibt sich ein besonders effizienter Betrieb der Anlage. Während für die Beheizung der Deethanizersäule Temperaturen von ca. 70 °C erforderlich sind, sind dies zur Verdampfung des Verdampfungseinsatzstroms, der auf nochmals geringerem Druck vorliegt, wie erwähnt beispielsweise bei ca. 17 bar, nur Temperaturen von ca. 50 °C. Dies ermöglicht es, zunächst Wärme in dem zum B eheizen der Destillationssäule verwendeten Wärmetauscher und anschließend in dem zum Verdampfen des Verdampfungseinsatzstroms verwendeten Wärmetauscher abzuführen. Die Wärme wird hierdurch besonders effizient genutzt.

Ein entsprechendes Verfahren kann entweder umfassen, dass der Verdampfungseinsatzstrom zusätzlich unter Verwendung von Fluid eines propanreichen Frischeinsatzstroms gebildet wird, und/oder dass in dem zum Verdampfen des Verdampfungseinsatzstroms verwendeten Wärmetauscher ein separater, propanreicher Frischeinsatzstrom verdampft wird. Auch zum Verdampfen eines propanreichen Frischeinsatzstroms muss daher keine extern bereitgestellte Wärme verwendet werden.

Besonders vorteilhafte Drücke im Rahmen der erläuterten Ausführungsform umfassen dabei, die Deethanizersäule auf einem Druckniveau von 25 bis 30 bar, insbesondere 27 bis 29 bar, zu betreiben, und den Verdampfungseinsatzstrom auf einem Druckniveau von 15 bis 20 bar, insbesondere von 16 bis 19 bar, zu verdampfen.

Wie bereits mehrfach erläutert, wird der Kondensatwasserstrom in dem Waschschritt auf einem Temperaturniveau von 90 bis 100 °C, beisp ielsweise bei ca. 94 °C, bereitgestellt. Weil es jedoch in typischen Verfahren zur Gewinnung von Propylen zu einem dynamischen Betrieb der zur Dehydrierung verwendeten Reaktoren sowie einer mit der Zeit variierenden Produktstromführung kommen kann, was bedeutet, dass die Menge an Dampf, die in der Wasserwäsche kondensiert werden muss, schwankt, kann es grundsätzlich zu einer Schwankung in der Temperatur des Kondensatwasserstroms kommen. Dies kann jedoch teilweise ausgeglichen werden, indem man Prozessdampf, der zeitweise nicht in der Dehydrierung benötigt und damit nicht in der Wasserwäsche kondensiert wird, an anderer Stelle gegen sich anwärmendes Kondensatwasser kondensiert. Der Kondensatwasserstrom kann also zusätzlich aufgewärmt werden, und zwar auf das zuvor erläuterte Temperaturniveau. Sicherheitshalber kann ferner vorgesehen sein, eine weitere, beispielsweise elektrische, Heizeinrichtung zum Backup entsprechender Temperaturschwankungen einzusetzen.

Eine erfindungsgemäße Anlage zur Gewinnung von Propylen umfasst Mittel, die im Einzelnen in dem entsprechenden unabhängigen Patentanspruch angegeben sind. Zu Merkmalen und Vorteilen einer entsprechenden Anlage sei auf die obigen Ausführungen verwiesen. Insbesondere ist eine entsprechende Anlage zur Durchführung eines Verfahrens eingerichtet, wie es zuvor im Detail erläutert wurde.

Die Erfindung wird nachfolgend unter Bezugnahme auf die beigefügten Zeichnungen näher erläutert, die bevorzugte Ausführungsformen der Erfindung unter Bezugnahme auf den Stand der Technik veranschaulichen.

### Kurze Beschreibung der Zeichnungen

Figur 1 veranschaulicht ein Verfahren zur Gewinnung von Propylen in Form eines schematischen Ablaufplans.
Figur 2 veranschaulicht Details eines Verfahrens zur Gewinnung von Propylen in Form eines schematischen Ablaufplans.
Figur 3 veranschaulicht Details eines Verfahrens zur Gewinnung von Propylen in Form eines schematischen Ablaufplans.
Figur 4 veranschaulicht Details eines Verfahrens zur Gewinnung von Propylen gemäß einer Ausführungsform der Erfindung in Form eines schematischen Ablaufplans.
Figur 5 veranschaulicht Details eines Verfahrens zur Gewinnung von Propylen gemäß einer Ausführungsform der Erfindung in Form eines schematischen Ablaufplans.

### Ausführliche Beschreibung der Zeichnungen

In den Figuren sind einander entsprechende Elemente, Anlagenteile und Ströme mit einander entsprechenden Bezugszeichen veranschaulicht und werden der Übersichtlichkeit halber nicht wiederholt erläutert.

In Figur 1 ist eine Anlage zur Gewinnung von Propylen in Form eines schematischen Ablaufplans veranschaulicht und insgesamt mit 100 bezeichnet.

In dem Verfahren 100 wird ein Einsatzstrom A einem Dehydrierungsschritt 101 unterworfen und in dem Dehydrierungsschritt zusammen mit einem Dampfstrom B zu einem Produktstrom C, der an dieser Stelle auch als Rohgasstrom bezeichnet werden kann, umgesetzt. Bei dieser Umsetzung wird ein Teil des in dem Einsatzstrom A enthaltenen Propans (der Einsatzstrom A ist reich an Propan oder besteht ausschließlich aus Propan) zu Propylen umgesetzt. Dem Dehydrierungsschritt 101 ist ein Katalysatorregenerationsschritt 102 zugeordnet, der der Übersichtlichkeit halber nicht erläutert wird. Zur Bereitstellung des Dampfstroms B ist ein Dampfbereitstellungsschritt 103 bzw. ein entsprechendes Dampferzeugungssystem vorgesehen. Dieses kann beispielsweise auch mit einem Kondensatwasserstrom B' gespeist werden, wie nachfolgend noch erläutert.

Der Produktstrom C (Rohgasstrom) wird einem Kühlschritt 104 unterworfen. Ein entsprechend abgekühlter Strom, nun mit E bezeichnet, wird anschließend einer Wasserwäsche (Quenchschritt) 105 zugeführt, in dem der Strom E in direkten Kontakt mit einem Wasserstrom Z gebracht wird. In der Wasserwäsche 105 wird zuvor in Form des Dampfstroms B zugeführter Prozessdampf teilweise aus dem Strom E auskondensiert. Ein hierbei erhaltener Kondensatwasserstrom wird beispielsweise in die Kondensatwasserströme B' und D aufgeteilt. Der Kondensatwasserstrom B' kann dem Dampferzeugungsschritt 103 unterworfen werden. Der Kondensatwasserstrom D wird abgekühlt und in Form eines Wasserstroms Z zum Kopf der Wasserwäsche 105 zurückgeführt. Ein von Wasser befreiter Strom, nun mit F bezeichnet, wird anschließend einem Verdichtungsschritt 106 unterworfen, der mit einem Sauergasentfernungsschritt 107 gekoppelt sein kann. Details werden nicht erläutert.

Nach der Verdichtung liegt ein hier als Trenneinsatzstrom bezeichneter Strom G vor und wird mehreren nachfolgenden Schritten zugeführt. Zunächst wird der Trenneinsatzstrom G dabei einem Vorkühl- und Trocknungsschritt 108 unterworfen, in welchem ein überwiegend Kohlenwasserstoffe mit zwei Kohlenstoffatomen, Methan und Wasserstoff enthaltender Strom H anfällt. Der Strom H enthält jedoch zudem noch Kohlenwasserstoffe mit drei und gegebenenfalls mehr Kohlenstoffatomen, welche in einem Niedertemperaturtrennschritt 109 in Form des Stroms K abgetrennt werden. Es verbleiben ein oder mehrere Restströme, wie hier mit O und P angegeben. Hierbei kann es sich beispielsweise um im Wesentlichen reinen Wasserstoff, jedoch auch um Gemische aus Wasserstoff und Methan und gegebenenfalls leichteren Kohlenwasserstoffen mit zwei Kohlenstoffatomen handeln. Die Restströme O und/oder P können zumindest teilweise zur Verdichtung zurückgeführt werden, wie im Folgenden noch erläutert wird.

Ein Strom I, der ebenfalls in dem Vorkühl- und Trocknungsschritt 108 anfällt und überwiegend Kohlenwasserstoffe mit zwei und mehr Kohlenstoffatomen enthält, wird zusammen mit dem erwähnten Strom K einem Deethanizerschritt 110 zugeführt. Der Deethanizerschritt dient im Wesentlichen zur Abtrennung eines an Kohlenwasserstoffen mit zwei Kohlenstoffatomen reichen und an Kohlenwasserstoffen mit drei und mehr Kohlenstoffatomen armen Stroms T, der hier erneut dem Vorkühl- und Trocknungsschritt 108 unterworfen wird. Ein verbleibender Strom S enthält überwiegend Kohlenwasserstoffe mit drei und mehr Kohlenstoffatomen und ist arm an Kohlenwasserstoffen mit zwei Kohlenstoffatomen, Methan und Wasserstoff.

Der Strom S wird einem Produkttrennschritt 111 zugeführt, in welchem im Wesentlichen Propylen in Form des Stroms U aus dem Strom S abgetrennt wird. Der Produkttrennschritt 111 ist hierzu mit einem Propylenkältemittelkreislauf 112 gekoppelt. Ein Strom V enthält überwiegend Kohlenwasserstoffe mit drei und mehr Kohlenstoffatomen, nachdem das Propylen in Form des Stroms U abgetrennt wurde. Der Strom V wird einem weiteren Trennschritt 113 zugeführt, in welchem Kohlenwasserstoffe mit vier und mehr Kohlenstoffatomen in Form des Stroms W aus dem Strom V abgetrennt werden. In dem weiteren Trennschritt 113 wird typischerweise auch ein propanreicher Strom X als sogenannter Frischfeed (Frischeinsatzstrom) zugespeist. Ein aus dem Strom V nach der Abtrennung des Stroms W und dem Strom X gebildeter Strom Y, der noch einen gewissen Anteil an Kohlenwasserstoffen mit drei und vier Kohlenstoffatomen in ungesättigter Form enthalten kann, wird einer Hydrierungseinheit 114 zugeführt. Stromab der Hydrierungseinheit 114 liegt der bereits erwähnte Strom A vor, bei dem es sich im Wesentlichen um reines Propan handelt.

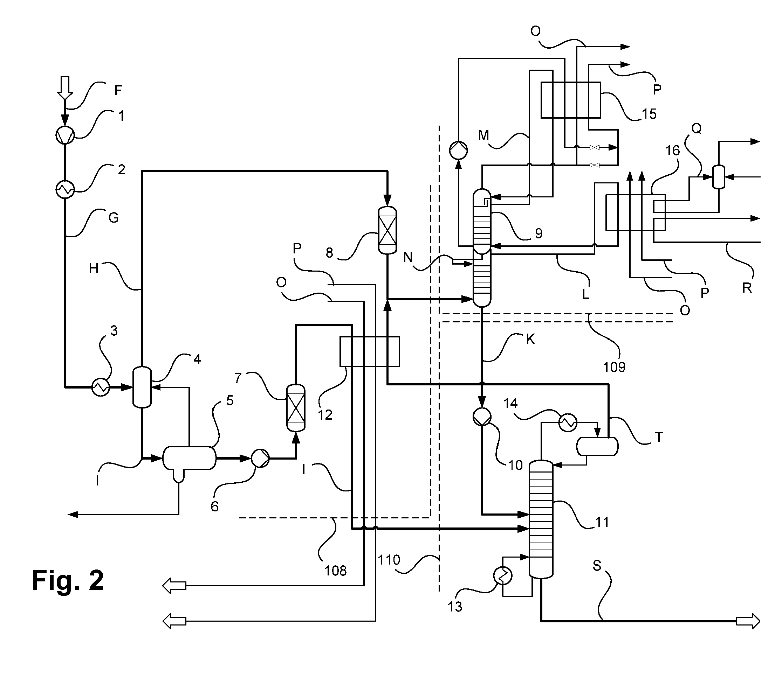

In Figur 2 sind Details eines Verfahrens zur Gewinnung von Propylen veranschaulicht, wobei in der Darstellung der Figur 2 insbesondere die Komponenten 108, 109 und 110 des in Figur 1 veranschaulichten Verfahrens 100, also der Vorkühl- und Trocknungsschritt 108, der Tieftemperaturtrennschritt 109 und der Deethanizerschritt 110 veranschaulicht sind. Die Trennung der einzelnen Schritte ist nicht durch die Darstellung in der Zeichnung festgelegt.

Der Trenneinsatzstrom G, der stromauf, wie erläutert, auf eine zur Trennung geeignete Temperatur abgekühlt und zuvor entsprechend verdichtet worden sein kann, wird im dargestellten Beispiel aus dem erwähnten Strom F gebildet und beispielsweise auf einem Druck von 28 bar und einer Temperatur von 37 °C, die mittels eines Verdichters 1 bzw. eines Wärmetauschers 2 erhalten wurden, einem weiteren Wärmetauscher 3 zugeführt. Der Strom G wird in dem weiteren Wärmetauscher 3 von der zuvor erläuterten Temperatur, die hier als erstes Temperaturniveau bezeichnet wird, auf eine Temperatur von beispielsweise +10 °C, die hier als zweites Temperaturniveau bezeichnet wird, abgekühlt. Zur Abkühlung in dem Wärmetauscher 3 wird beispielsweise Propan als Kältemittel verwendet.

Der entsprechend abgehkühlte Strom wird anschließend in einen Abscheiderbehälter 4 überführt, in welchem ein "erster" flüssiger Kondensatstrom in Form des Stroms I sowie ein "erster" gasförmiger Reststrom in Form des Stroms H erhalten werden. Die Ströme I und H sind bereits in der Figur 1 veranschaulicht. Aus dem Strom I wird in einem Wasserabscheider 5 Wasser abgeschieden (der Wasserstrom ist nicht gesondert bezeichnet) und der Strom I wird anschließend mittels einer Pumpe 6 einem adsorptiven Trockner 7 zugeführt. Entsprechend wird auch der Strom H einem adsorptiven Trockner 8 zugeführt. Der Strom H wird anschließend in eine Doppelsäule 9 überführt, in welcher der bereits zuvor erläuterte Strom K erhalten und mittels einer Pumpe 10 in eine Deethanizersäule 11 überführt wird.

Der Deethanizersäule 11 wird ferner der Strom I zugeführt, der nach der Trocknung in dem adsorptiven Trockner 7 in einem Wärmetauscher 12 angewärmt wurde. In der Deethanizersäule 11, die einen Sumpfverdampfer 13 und einen Kopfkondensator 14 aufweist, wird bodenseitig der bereits mehrfach erläuterte Strom S gewonnen, also ein Strom, der überwiegend Kohlenwasserstoffe mit drei und mehr Kohlenstoffatomen aufweist. Kopfseitig wird ein gasförmiger Strom abgezogen. Ein nicht in dem Kopfkondensator 14 kondensierender Anteil hiervon wird in Form des ebenfalls erläuterten Stroms T nach Abkühlung in Wärmetauscher 12 mit dem Strom H vereinigt. Die Deethanizersäule 11 wird beispielsweise im Sumpf bei ca. 70 °C und einem Druck von 28 bar und in dem Kopfkondensator 14 bei einer Temperatur von 37 °C betrieben.

Vom Kopf des oberen bzw. unteren Teils der Destillationssäule 9 werden jeweils Ströme L und M abgezogen und in Wärmetauschern 15 bzw. 16 teilverflüssigt. Der Strom L wird in den oberen Teil der Destillationssäule 9 eingespeist, der Strom M am Kopf des oberen Teils der Destillationssäule 9 aufgegeben. Aus dem Sumpf des oberen Teils der Destillationssäule 9 wird ein Strom N abgezogen und in den unteren Teil der Destillationssäule 9 überführt. Aus dem oberen Teil der Destillationssäule 9 werden ferner gasförmige Ströme abgezogen, durch den Wärmetauscher 15 geführt und als Tailgasstrom O bzw. als Recyclestrom P ausgeführt. Der Tailgasstrom O kann beispielsweise überwiegend Wasserstoff enthalten, der Recyclestrom P beispielsweise Kohlenwasserstoffe mit zwei Kohlenstoffatomen, Methan und Wasserstoff.

Im dargestellten Beispiel wird der Wärmetauscher 16 mittels zweier Kältekreisläufe Q und R betrieben, wobei es sich bei dem Kältekreislauf Q um einen Kältekreislauf mit Propen auf einer Temperatur von -20 °C und bei dem Kältekreislauf R um einen Kältekreislauf mit Propen auf -40 °C handeln kann. Zusätzlich werden durch den Wärmetauscher 16 der Recyclestrom O und der Tailgasstrom P geführt. Recyclestrom O und der Tailgasstrom P werden ferner in Wärmetauscher 12 weiter angewärmt.

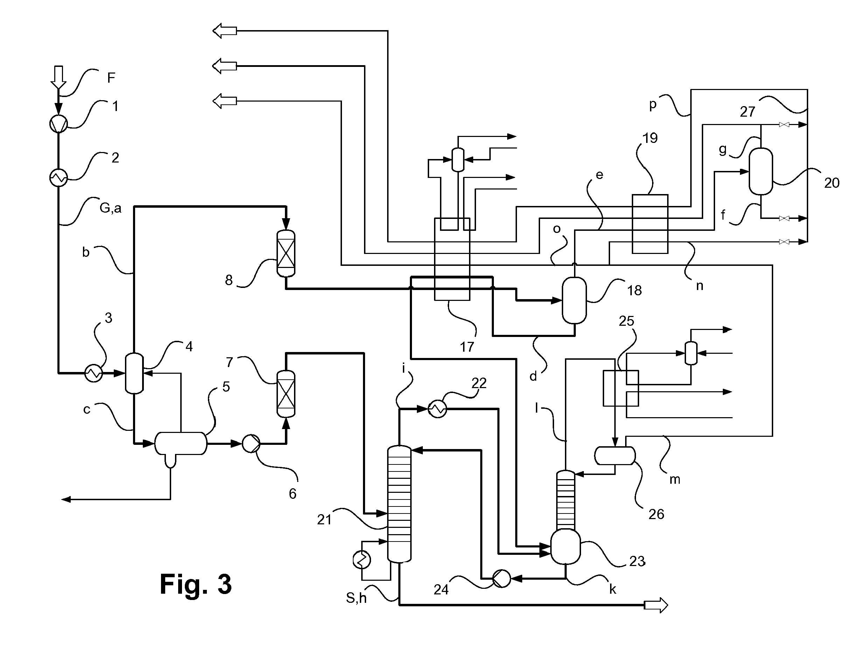

In Figur 3 sind Details eines alternativen Verfahrens zur Gewinnung von Propylen schematisch veranschaulicht. Zur besseren Unterscheidbarkeit sind hier die jeweiligen Ströme mit Kleinbuchstaben bezeichnet.

Ein Trenneinsatzstrom G, der hier auch mit a bezeichnet wird, wird ebenfalls durch einen Verdichter 1 und einen Wärmetauscher 2 geführt. Wie zuvor liegt dieser anschließend auf dem ersten Temperaturniveau vor. Auch hier wird dieser Strom durch einen weiteren Wärmetauscher 3 geführt und anschließend in einen Abscheiderbehälter 4 auf dem zweiten Temperaturniveau eingespeist. Auch die Abscheidung von Wasser aus dem flüssigen Kondensatstrom, der hier mit c bezeichnet ist ("erster" Kondensatstrom, vergleiche Strom I gemäß Figur 2) in dem Wasserabscheider 5 erfolgt in vergleichbarer Weise, ebenso wie die zunächst weitere Behandlung des Stroms c in der Pumpe 6 und dem adsorptiven Trockner 7. Entsprechendes gilt auch für den hier mit b bezeichneten Restgasstrom ("erster" Restgasstrom, vergleiche Strom H gemäß Figur 2). Der Strom b wird hier jedoch anschließend durch einen Wärmetauscher 17 geführt und, nach Abkühlung auf ein drittes Temperaturniveau, das beispielsweise bei -37 °C liegt, in einen weiteren Abscheiderbehälter 18 überführt. Der Druck an dieser Stelle beträgt beispielsweise 27 bar. In dem Abscheiderbehälter 18 wird ein "zweiter" flüssiger Kondensatstrom d gewonnen, der in dem Wärmetauscher 17 als Kältemittel verwendet wird. Der Wärmetauscher 17 kann, ähnlich wie der Wärmetauscher 25 bzw. der Wärmetauscher 15 gemäß Figur 2, mit zwei Propankältemittelkreisläufen betrieben werden. Am Kopf des Abscheiderbehälters 18 fällt ein "zweiter" gasförmiger Reststrom e an, der, ausgehend von dem dritten Temperaturniveau, in einem weiteren Wärmetauscher 19 auf ein viertes Temperaturniveau abgekühlt wird. Das vierte Temperaturniveau liegt beispielsweise bei -120 °C, der Druck an dieser Ste IIe beträgt beispielsweise ca. 27 bar. Nach Abkühlen auf das vierte Temperaturniveau wird der Strom e in einen weiteren Abscheiderbehälter 20 überführt, in welchem ein "dritter" flüssiger Kondensatstrom f und ein "dritter" gasförmiger Reststrom g erhalten werden.

Zunächst wird nun die weitere Trennung gemäß Figur 3 erläutert. Der Strom c, also der erste flüssige Kondensatstrom, wird dabei nach Abtrennung des Wassers in dem Wasserabscheider 5 und weiterer Trocknung in dem adsorptiven Trockner 7 in eine Deethanizersäule 21 überführt, in der Fluid des Stroms c aufgetrennt wird. Die Deethanizersäule 21 wird mit einem nicht näher bezeichneten Sumpfverdampfer und beispielsweise auf einer Temperatur von 70 °C und einem Druck von 28 bar betrieben.

Im Sumpf der Deethanizersäule 21 fällt ein Sumpfprodukt an, das in Form eines Propylen und Propan enthaltenden, an Kohlenwasserstoffen mit zwei Kohlenstoffatomen, Methan und Wasserstoff armen, jedoch gegebenenfalls noch Kohlenwasserstoffe mit vier und mehr Kohlenstoffatomen enthaltenden Stroms h aus der Deethanizersäule 21 abgezogen wird. Der Strom h entspricht damit im Wesentlichen dem Strom S gemäß den Figuren 1 und 2 und wird hier wie oben auch als "erster Trennabstrom" bezeichnet.

Vom Kopf der Deethanizersäule 21 wird ein Strom i abgezogen, durch einen Wärmetauscher 22 geführt und hier auf eine Temperatur von beispielsweise 37 °C abgekühlt. Der Strom i wird anschließend in eine zweite Destillationssäule 23 überführt, in welche auch der zweite flüssige Kondensatstrom d aus dem Abscheiderbehälter 18 eingespeist wird. Aus dem Sumpf der zweiten Destillationssäule 23 wird ein Strom k abgezogen und mittels einer Pumpe 24 in die Deethanizersäule 21 überführt.

Vom Kopf der zweiten Destillationssäule 23 wird ein gasförmiger Strom I abgezogen, durch einen Wärmetauscher 25 geführt, der mit Propankältemittel wie zuvor unter Bezugnahme auf den Wärmetauscher 15 der Figur 2 beschrieben gekühlt wird. In einem Abscheiderbehälter 26 eines hierdurch gebildeten Kopfkondensators der zweiten Destillationssäule 23 wird ein flüssiger Strom gewonnen und als Rücklauf auf die zweite Destillationssäule 23 zurückgeführt. Ein in dem Kopfkondensator bei beispielsweise ca. -31 °C und 26 bar gasförmig verb leibendes Fluid wird in Form des Stroms m bereitgestellt.

Ein Teil des Stroms m, der aufgrund seiner Bildung aus dem ersten flüssigen Kondensatstrom c und dem zweiten flüssigen Kondensatstrom d vergleichsweise geringe Mengen an Wasserstoff enthält, jedoch reich an Kohlenwasserstoffen mit zwei Kohlenstoffatomen ist, wird in Form des Stroms n abgezweigt, der Rest in Form des Stroms o durch den Wärmetauscher 17 geführt und als entsprechender Hochdruckstrom bereitgestellt.

Der dritte flüssige Kondensatstrom aus dem Sumpf des Abscheiderbehälters 20, ein Teil des dritten gasförmigen Reststroms g vom Kopf dieses Abscheiderbehälters 20 und der Strom n werden in eine Sammelleitung 27 entspannt, wodurch ein gemischtes Kältemittel in der Sammelleitung 27 erhalten wird. Dieses gemischte Kältemittel wird in Form des Stroms p durch den Wärmetauscher 19 zurückgeführt. Entsprechend wird der Rest des gasförmigen Reststroms vom Kopf des Abscheiderbehälters 20 durch den Wärmetauscher 19 geführt.

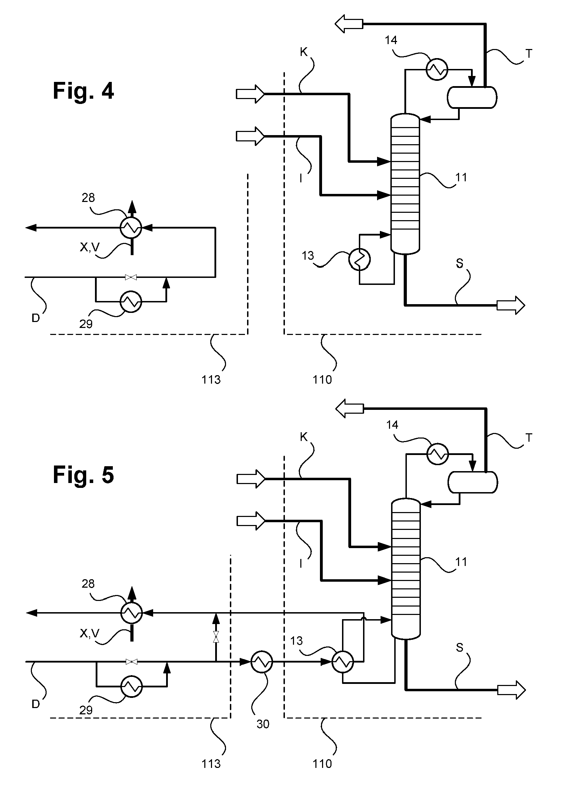

In Figur 4 sind Details eines weiteren, zum Vergleich mit der Erfindung dargestellten Verfahrens veranschaulicht, wobei die Figur 4 insbesondere Details des Verfahrensschritts 110, d.h. des Deetanizerschritts, und des Verfahrensschritts 113, d.h. des weiteren Trennschritts, und hier nur die Verdampfung eines entsprechenden Stroms, zeigt.

Der Verfahrensschritt 110 umfasst, wie erläutert, die Verwendung einer Deethanizersäule, die einen bereits in den Figuren 2 und 3 veranschaulichten Sumpfverdampfer aufweist. Die Darstellung der Figur 4 und auch der nachfolgenden Figur 5 entspricht dabei insoweit der Figur 2, als hier eine Deethanizersäule 11 mit einem Sumpfverdampfer 13 und die Einspeisung der erläuterten Ströme I und K in diese Deethanizersäule 11 gezeigt ist. Die Erfindung eignet sich jedoch in gleicher Weise in Verwendung mit einer Deethanizersäule 21 gemäß Figur 3.

Der Sumpfverdampfer 13 wird in dem in Figur 4 dargestellten Verfahren typischerweise mit Niederdruckdampf beheizt. Die Destillationssäule 11 wird typischerweise bei einer Temperatur von ca. 70 °C und einem Druck von ca. 28 bar betrieben. Der Kopfkondensator der Destillationssäule 9 wird typischerweise, wie ebenfalls erwähnt, auf einer Temperatur von ca. 37 °C betrieben.

In dem in Figur 4 veranschaulichten Verfahren wird in dem Deethanizerschritt 110 keine Wärme eines Kondensatwasserstroms D eingesetzt. Der Kondensatwasserstrom D dient im dargestellten Beispiel, wie in Verfahrensschritt 113 erläutert, zur Beheizung und damit Verdampfung des Stroms X und/oder des Stroms V. Zur Beheizung bzw. Verdampfung kommt ein Wärmetauscher 28 zum Einsatz. Ein weiterer Wärmetauscher 29, der wahlweise mit einem einstellbaren Anteil des Kondensatwasserstroms D durchströmt werden kann, kann zur Abkühlung von überschüssigem Prozessdampf des Dampfstroms B verwendet werden, wenn dieser in dem Dehydrierschritt 101 in geringerer Menge benötigt wird. Hierdurch können Schwankungen in der Temperatur des Kondensatwasserstroms ausgeglichen werden, indem der Kondensatwasserstrom D hier zusätzlich beheizt wird. Der Kondensatwasserstrom D kann beispielsweise auf einem Temperaturniveau von 75 bis 100 °C, insbesond ere von 78 bis 94 °C, bereitgestellt werden. Stromab des Wärmetauschers 28 liegt die Temperatur des Kondensatwasserstroms typischerweise bei 85°C. Der Kondensatwasserstrom D wird auf diesem Temperaturniveau beispielsweise einer Luftkühlung oder Wasserkühlung zugeführt und in Form des Wasserstroms Z (siehe Figur 1) zum Kopf der Wasserwäsche 105 zurückgeführt.

In Figur 5 sind Details eines Verfahrens gemäß einer Ausführungsform der Erfindung veranschaulicht, wobei ebenfalls die Verfahrensschritte 100 und 113, wie bereits mehrfach erläutert, veranschaulicht sind. Details bezüglich der Destillationssäule 11, ihres Sumpfverdampfers 13 und ihres Kopfkondensators 14 sowie der Ströme I, K, S, T und D wurden bereits erläutert.

In dem in Figur 5 dargestellten Beispiel wird jedoch der Sumpfverdampfer 13 der Deethanizersäule 11 nicht mittels Niederdruckdampf, wie unter Bezugnahme auf die Figur 4 erläutert, sondern ebenfalls mit dem Kondensatwasserstrom D betrieben. Die Wärmetauscher 28 und 29 wurden bereits zuvor erläutert, für die Temperatur der Bereitstellung des Kondensatwasserstroms D gilt ebenso das zuvor Erläuterte. Vor dem Führen durch den Wärmetauscher 13 sollte der Kondensatwasserstrom D vorteilhafterweise eine Temperatur von ca. 94 °C au fweisen, damit eine ausreichende Temperaturdifferenz in dem Wärmetauscher 13 sichergestellt ist. Er wird daher ggf. zusätzlich in Wärmetauscher 29 (wie zuvor erläutert) sowie in Heizer 30, wie im Nachfolgendem erläutert, beheizt.

Zusätzlich ist in der Figur 4 ein weiterer Heizer 30 dargestellt, der zum Einsatz kommen kann, wenn der Kondensatwasserstrom D keine ausreichende Temperatur aufweist. Vorteilhafterweise weist der Kondensatwasserstrom D stromauf des Sumpfverdampfers 13 der Deethanizersäule 11 eine Temperatur von ca. 94°C auf, stromab dieser eine Temperatur von ca. 86 °C. Auf dem entsprechend verringerten Temperaturniveau durchläuft der Kondensatwasserstrom D den Wärmetauscher 28, wobei auch eine derart verringerte Temperatur zur Verdampfung eines entsprechenden Stroms V und/oder X ausreicht.

## Patentansprüche

1. Verfahren (100) zur Gewinnung von Propylen, bei dem
- aus Fluid eines Propan enthaltenden Dehydrierungseinsatzstroms (A) und eines Wasserdampfstroms (B) in einem Dehydrierungsschritt (101) ein Wasserdampf, Wasserstoff sowie Kohlenwasserstoffe mit einem bis vier Kohlenstoffatomen, darunter Propylen, enthaltender, erwärmter Dehydrierungsabstrom (C) gebildet wird,
- Fluid des Dehydrierungsabstroms (C) in einem Waschschritt (105) in direkten Kontakt mit einem Wasserstrom (Z) gebracht und abgekühlt und dabei ein erwärmter und überwiegend Wasserdampf des Dehydrierungsabstroms (C) in kondensierter Form und Wasser des Wasserstroms (Z) enthaltender Kondensatwasserstrom (D) sowie ein an Wasserdampf abgereicherter, zumindest Wasserstoff und Kohlenwasserstoffe mit einem bis vier Kohlenstoffatomen enthaltender Waschabstrom (F) gebildet wird, und
- zumindest unter Verwendung von Fluid des Waschabstroms (F) ein Kohlenwasserstoffe mit drei und vier Kohlenstoffatomen enthaltender, an Wasserstoff, Kohlenwasserstoffen mit einem und zwei Kohlenstoffatomen und Propylen armer, flüssiger Verdampfungseinsatzstrom (V) gebildet und verdampft wird, wobei das zu Bildung des Verdampfungseinsatzstroms (V) verwendete Fluid des Waschabstroms (F) unter Verwendung einer Deethanizersäule (11, 21) gebildet wird, in die zumindest ein aus dem Waschabstrom (F) gebildetes Fluid eingespeist wird,
**dadurch gekennzeichnet, dass**
- zur Beheizung eines Sumpfs der Deethanizersäule (11, 21) und zur Verdampfung des Verdampfungseinsatzstroms (V) Wärme des Kondensatwasserstroms (D) verwendet wird.

2. Verfahren nach Anspruch 1, bei dem zum Verdampfen des Verdampfungseinsatzstroms (V) und zum Beheizen der Deethanizersäule (11, 21) jeweils ein Wärmetauscher (28, 13) verwendet wird.

3. Verfahren nach Anspruch 2, bei dem Fluid des Kondensatwasserstroms (D) zunächst durch den zum Beheizen der Deethanizersäule (11, 21) verwendeten Wärmetauscher (13) und danach durch den zum Verdampfen des Verdampfungseinsatzstroms (V) verwendeten Wärmetauscher (28) geführt wird.

4. Verfahren nach einem der vorstehenden Ansprüche, bei dem der Verdampfungseinsatzstrom (V) ferner unter Verwendung von Fluid eines propanreichen Frischeinsatzstroms gebildet wird.

5. Verfahren nach Anspruch 2 oder 3, bei dem in dem zum Verdampfen des Verdampfungseinsatzstroms (V) verwendeten Wärmetauscher (28) ein separater, propanreicher Frischeinsatzstrom (X) verdampft wird.

6. Verfahren nach einem der vorstehenden Ansprüche, bei dem die Deethanizersäule (11, 21) auf einem Druckniveau von 20 bis 30 bar, insbesondere 27 bis 29 bar, betrieben und der Verdampfungseinsatzstrom (V) auf einem Druckniveau von 15 bis 20 bar, insbesondere 16 bis 18 bar, verdampft wird.

7. Verfahren nach einem der vorstehenden Ansprüche, bei dem der Kondensatwasserstrom (D) in dem Waschschritt (105) auf einem Temperaturniveau von 80 bis 100 °C bereitgestellt oder auf einem tieferen Temperaturniveau bereitgestellt und anschließend weiter auf das Temperaturniveau von 80 bis 100 °C erwärmt wird.

8. Anlage zur Gewinnung von Propylen, mit Mitteln, die dazu eingerichtet sind,
- aus Fluid eines Propan enthaltenden Dehydrierungseinsatzstroms (A) und eines Wasserdampfstroms (B) in einem Dehydrierungsschritt (101) einen Wasserdampf, Wasserstoff sowie Kohlenwasserstoffe mit einem bis vier Kohlenstoffatomen, darunter Propylen, enthaltenden, erwärmten Dehydrierungsabstrom (C) zu bilden,
- Fluid des Dehydrierungsabstroms (C) in einem Waschschritt (105) in direkten Kontakt mit einem Wasserstrom (Z) zu bringen und abzukühlen und einen erwärmten und überwiegend Wasserdampf des Dehydrierungsabstroms (C) in kondensierter Form und Wasser des Wasserstroms (Z) enthaltenden Kondensatwasserstrom (D) sowie einen an Wasserdampf abgereicherten, zumindest Wasserstoff und Kohlenwasserstoffe mit einem bis vier Kohlenstoffatomen enthaltenden Waschabstrom (F) zu bilden, und
- zumindest unter Verwendung von Fluid des Waschabstroms (F) einen Kohlenwasserstoffe mit drei und vier Kohlenstoffatomen enthaltenden, an Wasserstoff, Kohlenwasserstoffen mit einem und zwei Kohlenstoffatomen und Propylen armen, flüssigen Verdampfungseinsatzstrom (V) zu bilden und zu verdampfen sowie das zu Bildung des Verdampfungseinsatzstroms (V) verwendete Fluid des Waschabstroms (F) unter Verwendung einer Deethanizersäule (11, 21) zu bilden, in die zumindest ein aus dem Waschabstrom (F) gebildetes Fluid eingespeist wird,
**gekennzeichnet durch** Mittel, die dazu eingerichtet sind,
- zur Beheizung eines Sumpfs der Deethanizersäule (11, 21) und zur Verdampfung des Verdampfungseinsatzstroms (V) Wärme des Kondensatwasserstroms (D) zu verwenden.

9. Anlage nach Anspruch 8, die zur Durchführung eines Verfahrens nach einem der Ansprüche 1 bis 7 eingerichtet ist.

10. Anlage nach Anspruch 8 oder 9, bei der die Mittel, die dazu eingerichtet sind, zur Beheizung eines Sumpfs der Deethanizersäule (11, 21) und zur Verdampfung des Verdampfungseinsatzstroms (V) Wärme des Kondensatwasserstroms (D) zu verwenden, einen Wärmetauscher (13) zum Beheizen der Deethanizersäule (11, 21) und einen Wärmetauscher (28) zum Verdampfen des Verdampfungseinsatzstroms (V) aufweisen, die in Strömungsrichtung des Kondensatwasserstroms (D) hintereinander angeordnet sind.
